# EUROPÄISCHE PATENTSCHRIFT

(11) **EP 0 824 512 B1**
(45) Veröffentlichungstag und Bekanntmachung des Hinweises auf die Patenterteilung: **20.12.2000**
(21) Anmeldenummer: 96915021.8
(22) Anmeldetag: 07.05.1996
(51) Int. Cl.: C07C 213/08, C07C 233/23, C07C 271/24, C07C 271/56

(54) **SYNTHESE VON OPTISCH AKTIVEM AMINOINDANOL**
SYNTHESIS OF OPTICALLY ACTIVE AMINOINDANOL
SYNTHESE D'AMINOINDANOL OPTIQUEMENT ACTIF

(30) Priorität: 12.05.1995 DE 19517421
(43) Veröffentlichungstag der Anmeldung: 25.02.1998
(73) Patentinhaber: BASF AKTIENGESELLSCHAFT, 67056 Ludwigshafen (DE)
(72) Erfinder: NÜBLING, Christoph, D-67454 Ha loch (DE); LADNER, Wolfgang, D-67136 Fussgönheim (DE)
(86) Internationale Anmeldenummer: EP9601884
(87) Internationale Veröffentlichungsnummer: WO9635660

(56) Entgegenhaltungen:
- WO-A-95/07880
- WO-A-95/08636
- JOURNAL OF MEDICINAL CHEMISTRY, Bd. 35, Nr. 10, 15.Oktober 1992, Seiten 1685-1701, XP000560996 THOMPSON W J ET AL: "SYNTHESIS AND ANTIVIRAL ACTIVITY OF A SERIES OF HIV-1 PROTEASE INHIBITORS WITH FUNCTIONALITY TETHERED TO THE P1 OR P1' PHENYL SUBSTITUENTS: X-RAY CRYSTAL STRUCTURE ASSISTED DESIGN" in der Anmeldung erwähnt
- JOURNAL OF THE AMERICAN CHEMICAL SOCIETY, Bd. 73, 1.Januar 1951, Seiten 1639-1641, XP000561140 LUTZ R E ET AL: "FURTHER STUDIES ON THE STABILITY OF -HYDROXYETHYLAMINES TOWARD THE OPPENAUER OXIDATION. CIS- AND TRANS-1-AMINO-2-INDANOLS"
- TETRAHEDRON, Bd. 47, Nr. 27, 1.Juli 1991, Seiten 4941-4958, XP000573999 DIDIER E ET AL: "CHEMO-ENZYMATIC SYNTHESIS OF 1,2- AND 1,3- AMINO-ALCOHOLS AND THEIR USE IN THE ENANTIOSELECTIVE REDUCTION OF ACETOPHENONE AND ANTI-ACETOPHENONE OXIME METHYL ETHER WITH BORANE"

## Beschreibung

(1S, 2R)-1-Aminoindan-2-ol (1) ist ein wichtiges Zwischenprodukt für HIV-Proteaseinhibitoren [*1*) I. Houpis et al., Tetrahedron Lett. 35 (1994) 9355; *2*) B. Kim et al., Tetrahedron Lett. 35 (1994) 5153; *3*) D. Askin et al., Tetrahedron Lett. 35 (1994) 673; *4*) B. Dorsey et al., J. Med. Chem. 37 (1994) 3443; *5*) EP 617968; *6*) S. Young et al., J. Med. Chem. 35 (1992) 1702; *7*) W. Thompson et. al., J. Med. Chem. 35 (1992) 1685; *8*) D. Askin et al., J. Org. Chem. 57 (1992) 2771; *9*) J.Huff, J. Med. Chem. 34 (1991) 2305; *10*) T. Lyle et al., J. Med. Chem. 34 (1991) 1228].

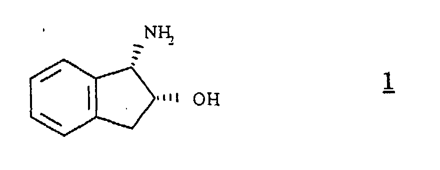

Verschiedene Synthesewege zu 1 wurden beschrieben: Racemisches *cis* 1-Aminoindan-2-ol kann durch Isomerisierung der entsprechenden trans Verbindung dargestellt werden, welche in zwei Stufen aus Inden erhalten werden kann [C. Suter et al., J. Am. Chem. Soc. 62 (1940) 3473; Lit. Ref. *7*)]. Die Racematspaltung wurde durch Chromatographie der diastereomeren Phenylalaninamide und deren Hydrolyse zu 1 [Lit. Ref. *7*)] oder durch Kristallisation der diastereomeren Tartrate [P. Reider (Merck & Co Inc) beim Kongress "Chiral '94" (Reston/Virginia, USA)] durchgeführt. Diese Methoden haben den Nachteil, daß Derivate für eine kostenträchtige Chromatographie oder eine aufwendige Rückführung der Weinsäure erforderlich sind.

In WO 95/08636 ist ein Verfahren zur Racematspaltung von *cis* und trans 1-Aminoindan-2-ol durch enzymatische Acylierung beschrieben, wobei nur *trans* 1-Aminoindan-2-ol in ausreichender Enantioselektivität acyliert wird. Das entstehende Amid kann direkt zur Isomerisierung verwendet werden. Nachteil dieser Methode ist die mangelnde chemische und thermische Stabilität sowie der hohe Preis der Enzyme.

In WO 95/07880 wird ein Verfahren zur Herstellung von enantiomerenreinem trans-1-Aminoindan-2-ol beschrieben. Ausgangsmaterial im Verfahren sind enantiomerenangereicherte trans-Benzamide oder trans-Acetamide des trans-1-Aminoindan-2-ol oder enantiomerenangereichtes trans-1-Aminoindan-2-ol. Diese enantiomerenangereichten Ausgangsmaterialien werden in Gegenwart eines chiralen Salen-Katalysators aus Inden über Oxydation mit wässrigem Hypochlorid hergestellt und im Falle der Amide anschließend mit Benzoylchlorid oder Acetanhydrid acyliert. Nach Kristallisation lassen sich die enantiomerenreinen Produkte aus diesen enantiomerenangereicherten Ausgangsmaterialen darstellen.

Durch asymmetrische Epoxidierung von Inden [P. van Eikeren (Sepracor Inc.) beim Kongress "Chiral '94" (Reston/Virginia, USA)] erhält man optisch aktives Indenepoxid (ee = 86%), aus dem in wenigen Stufen 1 erhalten werden kann. Aufgrund des ungenügenden Enantiomerenüberschusses muß hier jedoch eine Anreicherung auf ee > 99,5% erfolgen, die durch Kristallisation des Benzamids des *trans* 1-Aminoindan-2-ols bzw. des *cis* 1-Aminoindan-2-ol-Tartrats erreicht werden kann. Bei diesem Verfahren sind jedoch zusätzliche Derivatisierungsschritte bzw. die Rückführung der Weinsäure und relativ große Mengen des Epoxidierungskatalysators erforderlich.

Eine weitere Darstellungsmöglichkeit von optisch reinem 1 wurde von E. Didier et al. [Tetrahedron Lett. 27 (1991) 4941] beschrieben Der entscheidende Schritt ist eine diastereo- und enantioselektive Reduktion von 1-Methoxycarbonylindan-2-on mit Bäckerhefe. Diese Synthese ist jedoch durch viele Stufen und geringe Ausbeuten gekennzeichnet.

Es bestand die Aufgabe, eine neue, kostengünstige Methode zur Darstellung von enantiomerenreinem (1S, 2R)-1-Aminoindan-2-ol (1) zu finden.

Die Racematspaltung durch Kristallisation ist auch heute noch die verbreitetste Methode zur Darstellung reiner Enantiomere. Sind geeignete funktionelle Gruppen vorhanden, so erfolgt die Spaltung meist durch Kristallisation diastereomerer Salze. In bestimmten Fällen ist jedoch auch eine direkte Kristallisation eines Enantiomeren möglich. Voraussetzung hierfür ist, daß eine Verbindung im festen Zustand als Konglomerat, also als 1 : 1 Mischung der beiden Enantiomeren vorliegt [Übersicht in: R. Sheldon, "Chirotechnology, industrial synthesis of optically active compounds", Dekker, 1993, S.173]. Solche direkten Kristallisationen werden z.B. bei der Herstellung von Chloramphenicol [P. Amiard, Experientia 15 (1959) 38] und α-Methyl-L-Dopa [D. Reinhold et al. J. Org. Chem. 33 (1968) 1209] angewandt bzw. sind z.B. zur Racematspaltung von Ibuprofen beschrieben [WO 93/14056].

Es ist vorteilhaft, ein Racemat auf einer möglichst frühen Synthesestufe in die Antipoden zu spalten, um möglichst wenig überflüssigen Ballast durch die Reaktionssequenz zu schleppen. Mit Ausnahme der vielstufigen Synthese von Didier et al. (s.o.) beginnen alle anderen Wege zu 1 mit Inden, das durch Epoxidierung und Öffnung des Epoxids mit Ammoniak leicht in *trans* 1-Aminoindan-2-ol überführt werden kann. Bisher wurden (mit Ausnahme des in WO 95/08636 angegebenen enzymatischen Verfahrens) keine Racematspaltungen von *trans* 1-Aminoindan-2-ol bzw. Derivaten davon beschrieben.

Überraschend wurde nun gefunden, daß die Racematspaltung auf der Stufe des *trans* 1-Aminoindan-2-ols durch direkte Kristallisation durchgeführt werden kann. Hierzu wird der racemische Aminoalkohol 2 mit Säuren bzw. Säurederivaten der allgemeinen Formel RCOX nach allgemein bekannten Methoden [Houben-Weyl, Methoden der Organischen Chemie, Thieme 1985, Bd E5, S. 934 ff] zu Amiden bzw. Carbamaten des Typs A umgesetzt.

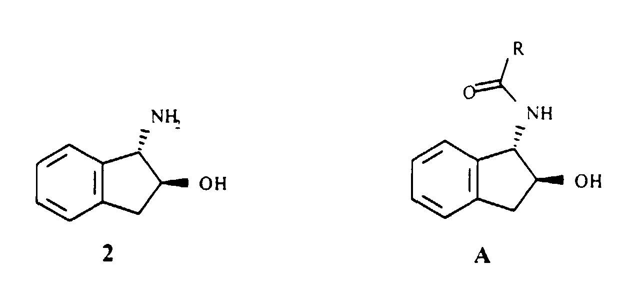

Der Substituent R hat folgende Bedeutungen: Wasserstoff, C₁-C₆-Alkyl, C₃-C₆-cycloalkyl, Phenyl, C₁-C₆-Alkoxy und Phenoxy, wobei diese Gruppen ein bis drei Halogenatome und/oder einen bis drei der folgenden Reste tragen können: Cyano, C₁-C₆-Alkoxy, C₁-C₆-Alkylthio, Phenyl, Phenylthio und Phenoxy, wobei die Phenylreste ihrerseits eine bis drei der folgenden Gruppen tragen können: C₁-C₄-Alkyl, C₁-C₄-Alkoxy, Halogen, Cyano und Nitro.

X bedeutet OH, Halogen, OR¹ und SR¹, wobei R¹ C₁-C₆-Alkyl, Phenyl und COR bedeutet.

Zur Racematspaltung wird eine übersättigte Lösung oder eine Schmelze eines racemischen Amids bzw. Carbamats A mit Impfkristallen des entsprechenden enantiomerenreinen Amids bzw. Carbamats versetzt, wodurch die Bildung von Kristallen induziert wird, die einen hohen Überschuß des Impfkristallenantiomeren enthalten. Die Kristalle werden abfiltriert und können durch Umkristallisation leicht in enantiomerenreiner Form erhalten werden. Eine der ausgefallenen Kristallmenge äquivalente Menge Racemat A wird im Filtrat, das nun einen Überschuß des anderen Enantiomeren enthält, durch Temperaturerhöhung gelöst. Durch Abkühlung wird die Lösung erneut übersättigt und anschließend mit Impfkristallen des anderen Enantiomeren versetzt, wodurch nun dieses selektiv auskristallisiert werden kann. Durch Wiederholung dieser Schritte (Lösen des Racemats im Filtrat, Übersättigung und abwechselnde Kristallisation der beiden Enantiomeren durch Zugabe von Impfkristallen) kann eine vollständige Racematspaltung eri reicht werden. Lediglich zu Beginn müssen auf unabhängigem Weg die Impfkristalle gewonnen werden, was z. B. durch die in WO 95/08636 beschriebene enzymatische Racematspaltung möglich ist.

Eine weitere Möglichkeit zur direkten Kristallisation eines Konglomerats besteht darin, eine übersättigte Lösung des Racemats parallel durch zwei Kristallisationsgefäße zu pumpen, in denen sich auf Filterböden Impfkristalle je eines der beiden Enantiomeren befinden. In einem Gefäß kristallisiert somit ein Teil des einen, im anderen Gefäß ein Teil des anderen Enantiomeren. Die Filtrate werden vereinigt und durch ein Vorratsgefäß, welches das Konglomerat enthält, geleitet, wo sie durch Erwärmen wieder gesättigt werden. Die gesättigte Lösung wird durch Abkühlung übersättigt und erneut in die Kristallisationsgefäße geleitet, wodurch sich der Kreislauf schließt [beispielhaft beschrieben in Chem. Eng. Nov. 8, 1965].

Als Lösungsmittel eignen sich Ether wie Tetrahydrofuran, Dioxan, Dimethoxyethan und Diglykoldimethylether; C₁ - C₆ -Alkohole wie Methanol, Ethanol, Propanol, Isopropanol, die isomeren Butanole, die isomeren Pentanole und Hexanol; Kohlenwasserstoffe wie Toluol, und Xylol und halogenierte Kohlenwasserstoffe wie Methylenchlorid, Chloroform, Dichlorethan und Chlorbenzol. Es können auch Gemische von Lösungsmitteln eingesetzt werden. Das erfindungsgemäße Verfahren ist jedoch auch ohne Verwendung von Lösungsmitteln in einer Kirstallschmelze durchführbar.

Für die Racematspaltung von trans 1-Aminoindan-2-ol durch direkte Kristallisation sind bevorzugt Amide bzw. Carbamate des Typs A geeignet, wobei der Rest R folgende Bedeutung hat: C₁-C₆-Alkyl, Phenyl, C₁-C₆-Alkoxy und Phenoxy, wobei diese Gruppen ein bis drei Halogenatome und/oder einen bis drei der folgenden Reste tragen können: Cyano, C₁-C₆-Alkoxy, Phenyl und Phenoxy, wobei die Phenylreste ihrerseits eine bis drei der folgenden Gruppen tragen können: C₁-C₄-Alkyl, C₁-C₄-Alkoxy, Halogen, Cyano und Nitro.

Besonders bevorzugt sind Amide bzw. Carbamate des Typs A, bei denen der Rest R folgende Bedeutung hat: C₁-C₄-Alkyl, Phenyl, C₁-C₄-Alkoxy und Phenoxy, wobei diese Gruppen ein bis drei Chloratome und/oder einen bis drei der folgenden Reste tragen können: C₁-C₄-Alkoxy, Phenyl und Phenoxy, wobei die Phenylreste ihrerseits eine bis drei der folgenden Gruppen tragen können: C₁-C₄-Alkyl, C₁-C₄-Alkoxy, Halogen, Cyano und Nitro.

Als besonders bevorzugt seien exemplarisch das Benzamid 3, das Methoxyacetamid 4, das Phenylpropionsäureamid 5, das Chloracetamid 6, das Methylcarbamat 7, das Phenylcarbamat 8 und das Benzylcarbamat 9 genannt.

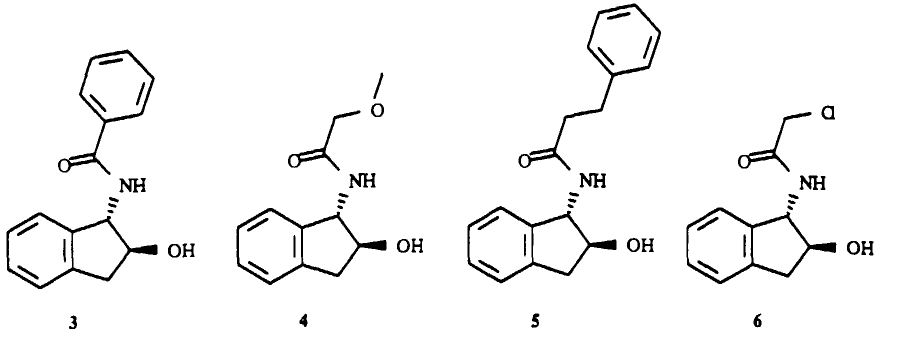

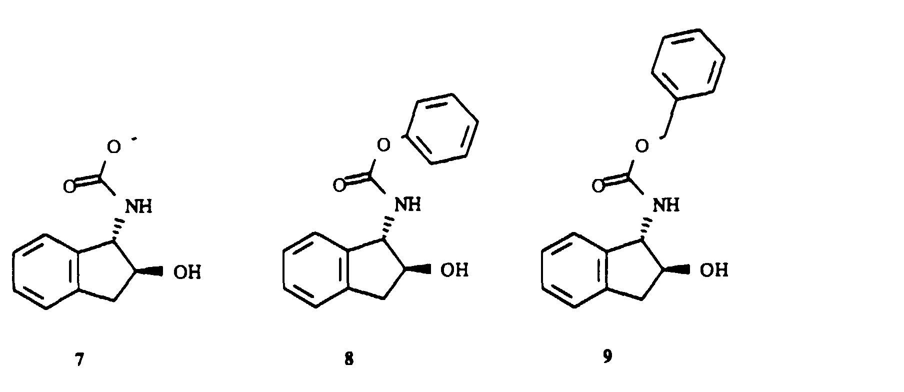

(1S, 2R)-1-Aminoindan-2-ol 1 wird aus den durch Kristallisation erhaltenen (1S, 2S)-Amiden bzw. (1S, 2S)-Carbamaten durch Umsetzung mit Thionylchlorid und saure Hydrolyse der entstehenden Oxazoline erhalten [R. Lutz et al., J. Am. Chem. Soc. 73 (1951) 1639]. Es sind also keine zusätzlichen Derivatisierungen erforderlich. Besonders vorteilhaft kann das Phenylpropionsäureamid 5 eingesetzt werden. Das enantiomerenreine Amid wird analog obiger Vorschrift mit Thionylchlorid in das entsprechende Oxazolin umgewandelt, welches alkalisch zum (1S, 2R)-1-Phenylpropionylamino-2-hydroxyindan hydrolysiert wird. Diese Verbindung ist die Folgestufe von 1 bei der Synthese von oral verfügbaren HIV-Proteaseinhibitoren (Lit. Ref. *3*).

Die Konzentration der übersättigten Lösungen ist abhängig vom Lösungsmittel, der Temperatur und dem relativen Verhältnis der beiden Enantiomeren. Sie liegt bei alkoholischen Lösungsmitteln typischerweise bei 1 - 10 Gew.%.

Die bei obigem Verfahren anfallenden Kristalle werden abfiltriert, mit kaltem Lösungsmittel nachgewaschen und getrocknet. Durch einmaliges Umkristallisieren erhält man praktisch enantiomerenreine Kristalle beider Enantiomeren (ee > 99,5%).

### Experimentelle Beispiele

### 1) Darstellung der Amide

a1) *trans* 1-Benzoylamino-2-hydroxyindan (3): Zu einer Lösung von racemischem *trans* 1-Amino-2-hydroxyindan (2 g; 13.4 mmol) und Triethylamin (1 eq.) in THF (38 ml) wurde eine Lösung von Benzoylchlorid (1 eq.) in THF (2 ml) zugetropft und die Mischung bei RT bis zur vollständigen Umsetzung des Amins nachgerührt. Das Lösungsmittel wurde am Rotationsverdampfer abdestilliert, der Rückstand mit IM Salzsäure versetzt und kräftig verrührt. Die unlöslichen Kristalle wurden abgesaugt und aus Methanol umkristallisiert. Ausbeute: 1,25 g (37%) *trans* 1-Benzoylamino-2-hydroxyindan.
   IR (KBr): 3309, 1641, 1579, 1549, 1491, 1473, 1332, 1314, 1075, 750, 684 cm⁻¹
a2) (1R, 2R)-1-Benzoylamino-2-hydroxyindan: Die Darstellung erfolgte ausgehend von enantiomerenreinem (1R, 2R)-1-Amino-2-hydroxyindan (2 g) analog Vorschrift a1). Ausbeute: 1,6 g (47%). Das IR-Spektrum (KBr) ist mit dem der racemischen Verbindung identisch.
b1) *trans* 1-Methoxyacetylamino-2-hydroxyindan (4): Zu einer Lösung von racemischem trans 1-Amino-2-hydroxyindan (20 g; 134 mmol) und Triethylamin (1 eq.) in THF (180 ml) wurde eine Lösung von Methoxyacetylchlorid (1 eq.) in THF (20 ml) zugetropft und die Mischung bei RT bis zur vollständigen Umsetzung des Amins nachgerührt. Das Lösungsmittel wurde am Rotationsverdampfer abdestilliert, der Rückstand mit IM Salzsäure versetzt und kräftig verrührt. Die unlöslichen Kristalle wurden abgesaugt und aus Isopropanol umkristallisiert. Ausbeute: 10,4 g (35%) *trans* 1-Methoxyacetylamino-2-hydroxyindan. IR (KBr): 3428, 3302, 1646, 1548, 1420, 1328, 1322, 1116, 1078, 751, 744 cm⁻¹
b2) (1S, 2S)-1-Methoxyacetylamino-2-hydroxyindan: Diese Verbindung wurde durch enzymatische Racematspaltung (wie in WO 95/08636 beschrieben) erhalten. Das IR-Spektrum (KBr) ist mit dem der racemischen Verbindung identisch.
c1) *trans* 1-Phenylpropionylamino-2-hydroxyindan (5): Zu einer Lösung von racemischem *trans* 1-Amino-2-hydroxyindan (2 g; 13.4 mmol) und Triethylamin (1 eq.) in THF (38 ml) wurde eine Lösung von Phenylpropionsäurechlorid (1 eq.) in THF (2 ml) zugetropft und die Mischung bei RT bis zur vollständigen Umsetzung des Amins nachgerührt. Das Lösungsmittel wurde am Rotationsverdampfer abdestilliert, der Rückstand mit IM Salzsäure versetzt und kräftig verrührt. Die unlöslichen Kristalle wurden abgesaugt und aus Isopropanol/Methanol umkristallisiert. Ausbeute: 2,1 g (55%) *trans* 1-Phenylpropionylamino-2-hydroxyindan. IR (KBr): 3329, 3279, 1640, 1554, 1497, 1455, 1347, 1078, 751, 695 cm⁻¹
c2) (1R, 2R)-1-Phenylpropionylamino-2-hydroxyindan: Die Darstellung erfolgte ausgehend von enantiomerenreinem (1R, 2R)-1-Amino-2-hydroxyindan (2 g) analog Vorschrift cl). Ausbeute: 2,0 g (53%). Das IR-Spektrum (KBr) ist mit dem der racemischen Verbindung identisch.
d1) *trans* 1-Chloracetylamino-2-hydroxyindan (6): Zu einer Lösung von racemischem *trans* 1-Amino-2-hydroxyindan (2 g; 13.4 mmol) und Triethylamin (1 eq.) in THF (38 ml) wurde eine Lösung von Chloracetylchlorid (1 eq.) in THF (2 ml) zugetropft und die Mischung bei RT bis zur vollständigen Umsetzung des Amins nachgerührt. Das Lösungsmittel wurde am Rotationsverdampfer abdestilliert, der Rückstand mit IM Salzsäure versetzt und mit Methyl tert. butylether extrahiert (3 mal). Die organische Phase wurde getrocknet (Na₂SO₄) und eingeengt. Der Rückstand wurde aus Methanol umkristallisiert. Ausbeute: 50 mg (1,5%) *trans* 1-Chloracetylamino-2-hydroxyindan. IR (KBr): 3411, 3268, 1641, 1558, 1407, 1313, 1268, 1077, 777, 750 cm⁻¹
d2) (1R, 2R)-1-Chloracetylamino-2-hydroxyindan: Die Darstellung erfolgte ausgehend von enantiomerenreinem (1R, 2R)-1-Amino-2-hydroxyindan (2 g) analog Vorschrift d1). Ausbeute: 0,9 g (30%). Das IR-Spektrum (KBr) ist mit dem der racemischen Verbindung identisch.
e1) *trans* 1-Methoxycarbonylamino-2-hydroxyindan (7): Racemisches *trans* 1-Amino-2-hydroxyindan (2 g; 13,4 mmol) wurde in einer Lösung von Natriumcarbonat (1,42 g; 1 eq.) in Wasser (20 ml) suspendiert und bei Raumtemperatur unter Rühren tropfenweise mit Chlorameisensäuremethylester (1,27 g; 1 eq.) versetzt. Nach Ende der Zugabe wurde noch ca. 15 min bei RT nachgerührt. Die Reaktionsmischung wurde mit 0,5 M Schwefelsäure angesäuert (pH = 1), der Feststoff abgesaugt, im Vakuumtrockenschrank getrocknet und aus Methanol umkristallisiert. Ausbeute: 0,75 g (27 %) trans 1-Methoxycarbonylamino-2-hydroxyindan. IR (KBr): 3443, 3307, 1694, 1554, 1329, 1308, 1262, 1079, 1050, 743 cm⁻¹
e2) (1R, 2R)-1-Methoxycarbonylamino-2-hydroxyindan: Die Darstellung erfolgte ausgehend von enantiomerenreinem (1R, 2R)-1-Amino-2-hydroxyindan (2 g) analog Vorschrift el). Ausbeute: 1,3 g (47%). Das IR-Spektrum (KBr) ist mit dem der racemischen Verbindung identisch.
f1) *trans* 1-Phenoxycarbonylamino-2-hydroxyindan (8): Racemisches *trans* 1-Amino-2-hydroxyindan (2 g; 13,4 mmol) wurde in einer Lösung von Natriumcarbonat (1,42 g; 1 eq.) in Wasser (20 ml) suspendiert und bei Raumtemperatur unter Rühren tropfenweise mit Chlorameisensäurephenylester (2,1 g; 1 eq.) versetzt. Nach Ende der Zugabe wurde noch ca. 15 min bei RT nachgerührt. Die Reaktionsmischung wurde mit 0,5 M Schwefelsäure angesäuert (pH = 1), der Feststoff abgesaugt, im Vakuumtrockenschrank getrocknet und aus Methanol umkristallisiert. Ausbeute: 1,2 g (33 %) *trans* 1-Phenoxycarbonylamino-2-hydroxyindan. IR (KBr): 1700, 1535, 1493, 1251, 1235, 1214, 1207, 1076, 744 cm⁻¹
f2) (1R, 2R)-1-Phenoxycarbonylamino-2-hydroxyindan: Die Darstellung erfolgte ausgehend von enantiomerenreinem (1R, 2R)-1-Amino-2-hydroxyindan (2 g) analog Vorschrift f1). Ausbeute: 1,0 g (28%). Das IR-Spektrum (KBr) ist mit dem der racemischen Verbindung identisch.
g1) *trans* 1-Benzyloxycarbonylamino-2-hydroxyindan (9): Racemisches *trans* 1-Amino-2-hydroxyindan (2 g; 13,4 mmol) wurde in einer Lösung von Natriumcarbonat (1,42 g; 1 eq.) in Wasser (20 ml) suspendiert und bei Raumtemperatur unter Rühren tropfenweise mit Chlorameisensäurebenzylester (2,3 g; 1 eq.) versetzt. Nach Ende der Zugabe wurde noch ca. 15 min bei RT nachgerührt. Die Reaktionsmischung wurde mit 0,5 M Schwefelsäure angesäuert (pH =1), der Feststoff abgesaugt, im Vakuumtrockenschrank getrocknet und aus Methanol umkristallisiert. Ausbeute: 0,75 g (20 %) *trans* 1-Benzyloxycarbonylamino-2-hydroxyindan. IR (KBr): 3281, 1690, 1563, 1330, 1304, 1266, 1082, 1056, 750, 724 cm⁻¹
g2) (1R, 2R)-1-Benzyloxycarbonylamino-2-hydroxyindan: Die Darstellung erfolgte ausgehend von enantiomerenreinem (1R, 2R)-1-Amino-2-hydroxyindan (2 g) analog Vorschrift gl). Ausbeute: 1,5 g (40%). Das IR-Spektrum (KBr) ist mit dem der racemischen Verbindung identisch.

### 2) Kristallisation von trans 1-Methoxyacetylamino-2-hydroxyindan

a1) Racemisches *trans* 1-Methoxyacetylamino-2-hydroxyindan 4 (5 g) wurde in Isopropanol (100 ml) durch Erwärmen gelöst und bis zur beginnenden Kristallisation langsam abgekühlt (43°C). Es wurde erneut bis zur vollständigen Lösung des Amids erwärmt (51°C) und die Lösung durch Abkühlen auf 49°C übersättigt. Bei dieser Temperatur wurden Impfkristalle des enantiomerenreinen (1S, 2S)-1-Methoxyacetylamino-2-hydroxyindan (475 mg) zugegeben und die Mischung unter Rühren langsam auf 45°C abgekühlt. Der Niederschlag wurde abgesaugt und mit wenig kaltem Isopropanol nachgewaschen.
   Ausbeute: 940 mg; ee = 89,5%.
a2) Die Mutterlauge von 2a1) wurde mit racemischem 4 (1 g) versetzt und bis zur vollständigen Lösung des Amids erhitzt (60°C). Die durch Abkühlung auf 52°C übersättigte Lösung wurde mit (1R, 2R)-1-Methoxyacetylamino-2-hydroxyindan (50 mg) versetzt und die Mischung unter Rühren langsam auf 48°C abgekühlt. Der Niederschlag wurde abgesaugt und mit wenig kaltem Isopropanol nachgewaschen.
   Ausbeute: 310 mg; ee = 95%
b1) Racemisches trans l-Methoxyacetylamino-2-hydroxyindan 4 (50 g) wurde in Isopropanol (800 ml) durch Erwärmen gelöst. Beim langsamen Abkühlen wurde die Lösung bei 57°C mit Impfkristallen des enantiomerenreinen (1S, 2S)-1-Methoxyacetylamino-2-hydroxyindan (5 g) versetzt und die Mischung unter Rühren langsam auf 47°C abgekühlt. Bei dieser Temperatur wurden die Kristalle abgesaugt, mit wenig kaltem Isopropanol nachgewaschen und im Vakuumtrockenschrank getrocknet. Ausbeute: 11,3 g; ee = 95,6%.
b2) In der Mutterlauge von 2b1) wurde racemisches 4 (12 g) durch Erwärmen gelöst. Beim langsamen Abkühlen wurde die Lösung bei 57°C mit Impfkristallen des enantiomerenreinen (1R, 2R)-1-Methoxyacetylamino-2-hydroxyindan (5 g) versetzt und die Mischung unter Rühren langsam auf 47°C abgekühlt. Bei dieser Temperatur wurden die Kristalle abgesaugt, mit wenig kaltem Isopropanol nachgewaschen und im Vakuumtrockenschrank getrocknet. Ausbeute: 14,5 g; ee = 97%.

## Patentansprüche

1. Verfahren zur Herstellung von enantiomerenreinem *cis* 1-Amino-2-hydroxyindan, dadurch gekennzeichnet, daß
a) racemisches *trans* 1-Aminoindan-2-ol mit Säuren bzw. Säurederivaten der allgemeinen Formel RCOX nach allgemein bekannten Methoden zu Amiden bzw. Carbamaten des Typs A umgesetzt wird, 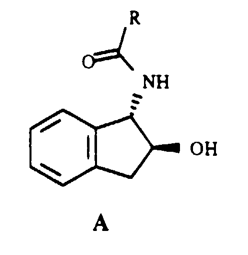 wobei die Substituenten folgende Bedeutungen haben:
R Wasserstoff, C₁-C₆-Alkyl, C₃-C₆-Cycloalkyl, Phenyl, C₁-C₆-Alkoxy und Phenoxy, wobei diese Gruppen ein bis drei Halogenatome und/oder einen bis drei der folgenden Reste tragen können: Cyano, C₁-C₆-Alkoxy, C₁-C₆-Alkylthio, Phenyl, Phenylthio und Phenoxy, wobei die Phenylreste ihrerseits eine bis drei der folgenden Gruppen tragen können: C₁-C₄-Alkyl, C₁-C₄-Alkoxy, Halogen, Cyano und Nitro.
X OH, Halogen, OR¹ und SR¹, wobei R¹ C₁-C₆-Alkyl, Phenyl und COR bedeutet.
b) eine übersättigte Lösung oder Schmelze des racemischen Amids bzw. Carbamats A mit Impfkristallen eines Enantiomeren versetzt wird,
c) die ausgefallenen Kristalle isoliert werden und die verbliebene Mutterlauge oder Schmelze nach Lösen von racemischem Amid bzw. Carbamat A erneut übersättigt und mit Impfkristallen des anderen Enantiomeren versetzt wird, wodurch dieses selektiv auskristallisiert wird.
d) das erhaltene enantiomerenreine trans Amid bzw. Carbamat nach bekannten Methoden zum enantiomerenreinen *cis* 1-Amino-2-hydroxyindan umgesetzt wird.

2. Verfahren nach Anspruch 1), dadurch gekennzeichnet, daß der Rest R folgende Bedeutung hat: C₁-C₆-Alkyl, Phenyl, C₁-C₆-Alkoxy und Phenoxy, wobei diese Gruppen ein bis drei Halogenatome und/oder einen bis drei der folgenden Reste tragen können: Cyano, C₁-C₆-Alkoxy, Phenyl und Phenoxy, wobei die Phenylreste ihrerseits eine bis drei der folgenden Gruppen tragen können: C₁-C₄-Alkyl, C₁-C₄-Alkoxy, Halogen, Cyano und Nitro.

3. Verfahren nach Anspruch 1), dadurch gekennzeichnet, daß der Rest R folgende Bedeutung hat: C₁-C₄-Alkyl, Phenyl, C₁-C₄-Alkoxy und Phenoxy, wobei diese Gruppen ein bis drei Chloratome und/oder einen bis drei der folgenden Reste tragen können: C₁-C₄-Alkoxy, Phenyl und Phenoxy, wobei die Phenylreste ihrerseits eine bis drei der folgenden Gruppen tragen können: C₁-C₄-Alkyl, C₁-C₄-Alkoxy, Halogen, Cyano und Nitro.

4. Verfahren nach Anspruch 1), dadurch gekennzeichnet, daß der Rest R Phenyl, Phenylethyl, Chlormethyl, Methoxymethyl, Methoxy, Phenoxy oder Benzyloxy bedeutet.

5. *trans* 1-Phenylpropionylamino-2-hydroxyindan sowie die beiden Enantiomeren (1S, 2S)- und (1R, 2R)-1-Phenylpropionylamino-2-hydroxyindan.

6. *trans* 1-Chloracetylamino-2-hydroxyindan sowie die beiden Enantiomeren (1S, 2S)- und (1R, 2R)-1-Chloracetylamino-2-hydroxyindan.

7. *trans* 1-Methoxycarbonylamino-2-hydroxyindan sowie die beiden Enantiomeren (1S, 2S)- und (1R, 2R)-1-Methoxycarbonylamino-2-hydroxyindan.

8. *trans* 1-Phenoxycarbonylamino-2-hydroxyindan sowie die beiden Enantiomeren (1S, 2S)- und (1R, 2R)-1-Phenoxycarbonylamino-2-hydroxyindan.

9. *trans* 1-Benzyloxycarbonylamino-2-hydroxyindan sowie die beiden Enantiomeren (1S, 2S)- und (1R, 2R)-1-Benzyloxycarbonylamino-2-hydroxyindan.

10. Verfahren nach Anspruch 1, bei dem als Lösungsmittel Ether, Alkohole, aromatische Kohlenwasserstoffe oder halogenierte Kohlenwasserstoffe verwendet werden.

## Claims

1. A process for preparing enantiomerically pure *cis*-1-amino-2-hydroxyindane, which comprises
a) converting racemic *trans*-1-aminoindan-2-ol with acids or acid derivatives of the general formula RCOX by conventional methods into amides or carbamates of type A 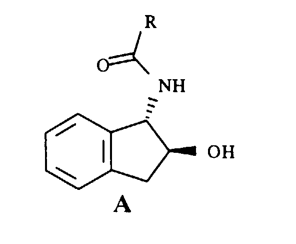 where the substituents have the following meanings:
R is hydrogen, C₁-C₆-alkyl, C₃-C₆-cycloalkyl, phenyl, C₁-C₆-alkoxy and phenoxy, it being possible for these groups to carry one to three halogen atoms and/or one to three of the following radicals: cyano, C₁-C₆-alkoxy, C₁-C₆-alkylthio, phenyl, phenylthio and phenoxy, it being possible for the phenyl radicals in turn to carry one to three of the following groups: C₁-C₄-alkyl, C₁-C₄-alkoxy, halogen, cyano and nitro,
X is OH, halogen, OR¹ and SR¹, where R¹ is C₁-C₆-alkyl, phenyl and COR,
b) adding seed crystals of one enantiomer to a supersaturated solution or melt of the racemic amide or carbamate A,
c) isolating the crystals which have separated out, and again supersaturating the remaining mother liquor or melt after dissolving racemic amide or carbamate A, and adding seed crystals of the other enantiomer, leading to selective crystallization of the latter,
d) converting the resulting enantiomerically pure trans amide or carbamate by conventional methods into the enantiomerically pure *cis*-1-amino-2-hydroxyindane.

2. A process as claimed in claim 1, wherein the radical R has the following meanings: C₁-C₆-alkyl, phenyl, C₁-C₆-alkoxy and phenoxy, it being possible for these groups to carry one to three halogen atoms and/or one to three of the following radicals: cyano, C₁-C₆-alkoxy, phenyl and phenoxy, it being possible for the phenyl radicals in turn to carry one to three of the following groups: C₁-C₄-alkyl, C₁-C₄-alkoxy, halogen, cyano and nitro.

3. A process as claimed in claim 1, wherein the radical R has the following meanings: C₁-C₄-alkyl, phenyl, C₁-C₄-alkoxy and phenoxy, it being possible for these groups to carry one to three chlorine atoms and/or one to three of the following radicals: C₁-C₄-alkoxy, phenyl and phenoxy, it being possible for the phenyl radicals in turn to carry one to three of the following groups: C₁-C₄-alkyl, C₁-C₄-alkoxy, halogen, cyano and nitro.

4. A process as claimed in claim 1, wherein the radical R is phenyl, phenylethyl, chloromethyl, methoxymethyl, methoxy, phenoxy or benzyloxy.

5. *trans*-1-Phenylpropionylamino-2-hydroxyindane and the two enantiomers (1S, 2S)- and (1R, 2R)-1-phenylpropionylamino-2-hydroxyindane.

6. *trans*-1-Chloroacetylamino-2-hydroxyindane and the two enantiomers (1S, 2S)- and (1R, 2R)-1-chloroacetylamino-2-hydroxyindane.

7. *trans*-1-methoxycarbonylamino-2-hydroxyindane and the two enantiomers (1S, 2S)- and (1R, 2R)-1-methoxycarbonylamino-2-hydroxyindane.

8. *trans*-1-phenoxycarbonylamino-2-hydroxyindane and the two enantiomers (1S, 2S)- and (1R, 2R)-1-phenoxycarbonylamino-2-hydroxyindane.

9. *trans*-1-benzyloxycarbonylamino-2-hydroxyindane and the two enantiomers (1S, 2S)- and (1R, 2R)-1-benzyloxycarbonylamino-2-hydroxyindane.

10. A process as claimed in claim 1, in which ethers, alcohols, aromatic hydrocarbons or halogenated hydrocarbons are used as solvents.

## Revendications

1. Procédé pour la 'préparation de cis 1-amino-2-hydroxyindane pur du point de vue énantiomère, caractérisé par le fait que
a) on convertit du trans 1-aminoindane-2-ol racémique avec des acides ou des dérivés d'acide de formule générale RCOX selon des méthodes connues de manière générale en amides ou carbamates du type A, 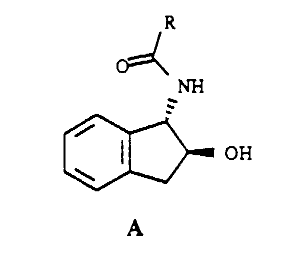 tandis que les substituants ont les significations suivantes:
R hydrogène, alkyle en C₁-C₆, cycloalkyle en C₃-C₆, phényle, alcoxy en C₁-C₆ et phénoxy, tandis que ces groupes peuvent porter un à trois atomes d'halogène et/ou un à trois des restes suivants: cyano, alcoxy en C₁-C₆, alkylthio en C₁-C₆, phényle, phénylthio et phénoxy, tandis que les restes phényle peuvent à leur tour porter un à trois des groupes suivants: alkyle en C₁-C₄, alcoxy en C₁-C₄, halogéno, cyano et nitro,
X OH, halogéno, OR¹ et SR¹, tandis que R¹ représente un groupe alkyle en C₁-C₆, phényle et COR,
b) on additionne une solution sursaturée ou des masses fondues de l'amide ou du carbamate racémique A avec des cristaux d'ensemencement d'un énantiomère,
c) on isole les cristaux formés et on sursature à nouveau la liqueur-mère ou la masse fondue restant après dissolution de l'amide ou du carbamate racémique A, et on additionne de cristaux d'ensemencement de l'autre énantiomère, ce qui amène à la cristallisation sélective de celui-ci,
d) on fait réagir le trans amide ou carbamate pur du point de vue énantiomère obtenu, selon des méthodes connues, pour former du cis 1-amino-2-hydroxyindane pur du point de vue énantiomère.

2. Procédé selon la revendication 1, caractérisé par le fait que le reste R a la signification suivante: alkyle en C₁-C₆, phényle, alcoxy en C₁-C₆ et phénoxy, tandis que ces groupes peuvent porter un à trois atomes d'halogène et/ou un à trois des restes suivants: cyano, alcoxy en C₁-C₆, phényle et phénoxy, tandis que les restes phényle peuvent à leur tour porter un à trois des groupes suivants: alkyle en C₁-C₄, alcoxy en C₁-C₄, halogéno, cyano et nitro.

3. Procédé selon la revendication 1, caractérisé par le fait que le reste R a la signification suivante: alkyle en C₁-C₄, phényle, alcoxy en C₁-C₄ et phénoxy, tandis que ces groupes peuvent porter un à trois atomes de chlore et/ou un à trois des restes suivants: alcoxy en C₁-C₄, phényle et phénoxy, tandis que les restes phényle peuvent à leur tour porter un à trois des groupes suivants: alkyle en C₁-C₄, alcoxy en C₁-C₄, halogéno, cyano et nitro.

4. Procédé selon la revendication 1, caractérisé par le fait que le reste R représente un groupe phényle, phényléthyle, chlorométhyle, méthoxyméthyle, méthoxy, phénoxy ou benzyloxy.

5. Trans 1-phénylpropionylamino-2-hydroxyindane, ainsi que les deux énantiomères (1S, 2S)- et (1R, 2R)-1-phénylpropionylamino-2-hydroxyindane.

6. Trans 1-chloroacétylamino-2-hydroxyindane, ainsi que les deux énantiomères (1S, 2S)- et (1R, 2R)-1-chloroacétylamino-2-hydroxyindane.

7. Trans 1-méthoxycarbonylamino-2-hydroxyindane, ainsi que les deux énantiomères (1S, 2S)- et (1R, 2R)-1-méthoxycarbonylamino-2-hydroxyindane.

8. Trans 1-phénoxycarbonylamino-2-hydroxyindane, ainsi que les deux énantiomères (1S, 2S)- et (1R, 2R)-1-phénoxycarbonylamino-2-hydroxyindane.

9. Trans 1-benzyloxycarbonylamino-2-hydroxyindane, ainsi que les deux énantiomères (1S, 2S)- et (1R, 2R)-1-benzyloxycarbonylamino-2-hydroxyindane.

10. Procédé selon la revendication 1, dans lequel on utilise comme solvant des éthers, des alcools, des hydrocarbures aromatiques ou des hydrocarbures halogénés.
